# FASCICULE DE BREVET EUROPEEN

(11) **EP 2 314 566 B1**
(45) Date de publication et mention de la délivrance du brevet: **24.01.2018**
(21) Numéro de dépôt: 10186984.0
(22) Date de dépôt: 08.10.2010
(51) Int. Cl.: C07C 221/00, C07D 311/88, C07F 7/08

(54) **Procédé de fonctionnalisation de molécules biologiques**
Verfahren zur Funktionalisierung von biologischen Molekülen
Process for the functionalization of biological molecules

(30) Priorité: 15.10.2009 FR 0957232
(43) Date de publication de la demande: 27.04.2011
(73) Titulaire: Commissariat à l'Énergie Atomique et aux Énergies Alternatives, 75015 Paris (FR)
(72) Inventeur: Bernardin, Aude, 45740 Lailly-en-Val (FR); Bonnaffe, David, 75004 Paris (FR); Texier-Nogues, Isabelle, 38000 Grenoble (FR)
(74) Mandataire: Nony

(56) Documents cités:
- WO-A2-2007/148089
- AGARD N J ET AL: "A strain-promoted [3 + 2] azide-alkyne cycloaddition for covalent modification of biomolecules in living systems", JOURNAL OF THE AMERICAN CHEMICAL SOCIETY, AMERICAN CHEMICAL SOCIETY, NEW YORK, US LNKD- DOI:10.1021/JA044996F, vol. 126, no. 46, 24 novembre 2004 (2004-11-24), pages 15046-15047, XP002362785, ISSN: 0002-7863
- HELMUT SEIDL, ROLF HUISGEN, RUDOLF KNORR: "Zur Anlagerung der Nitrone an C-C-Dreifachbindungen", CHEM. BER., no. 102, 21 août 1968 (1968-08-21), pages 904-914, XP002582975,
- A. CHATTERJEE ET AL: "water Exclusion reaction in Aqueous Media: Nitrone Formation and Cycloaddition in a Single Pot", ORG. LETT., vol. 5, no. 21, 17 septembre 2003 (2003-09-17), pages 3967-3969, XP002582976,

## Description

La présente invention vise à proposer une nouvelle méthode de couplage, particulièrement utile pour la fonctionnalisation de molécules biologiques comme par exemple les protéines, enzymes, acides nucléiques, dans la mesure où elle est réalisable en milieu aqueux et à température ambiante.

Les réactions de couplage sont une voie de fonctionnalisation largement utilisée notamment dans le domaine de la chimie au regard de leurs grandes spécificités.

En effet, en reposant sur l'interactivité de deux groupements spécifiques, portés respectivement par les deux entités que l'on cherche à coupler, ces réactions permettent de réduire significativement le risque de réaction(s) secondaire(s) et garantissent généralement un rendement satisfaisant en produit de couplage attendu.

La réalisation de cette réaction de couplage est toutefois généralement subordonnée, outre à la mise en présence des composés porteurs des deux groupements réactifs, à l'ajustement de paramètre(s) annexe(s). Ces paramètres peuvent être des paramètres physiques tels que température, pression atmosphérique, pH voire exposition à un rayonnement UV par exemple ou être de nature chimique tel que présence d'un catalyseur.

C'est notamment au regard de la nécessité de considérer certains des paramètres évoqués ci-dessus, qu'il existe assez peu de réactions de couplage utilisables en conditions biologiques (*in vitro* ou *in vivo*).

Pour des raisons évidentes, certains paramètres ne sont pas compatibles avec une mise en oeuvre en milieu biologique à l'image par exemple d'un pH très acide.

De plus, une réaction de couplage réalisée en milieu biologique ou à l'égard d'une molécule biologique impose des contraintes annexes : aucun des réactifs ou produits de la réaction ne doit être toxique; les produits et réactifs doivent être inertes biologiquement vis-à-vis des groupes fonctionnels du vivant : acides carboxyliques, amines, thiols, ... pour obtenir une meilleur spécificité de couplage ; la réaction doit avoir lieu rapidement en milieu aqueux, à pH et température physiologiques et, si possible, ne doit pas dépendre de la concentration en sels présents dans le milieu (Na, Cl, Mg, K, ...) et la réaction doit être efficace à faible concentration.

Comme réactions de couplage réalisable avec des molécules de nature biologique peuvent toutefois être citées celles reposant sur l'interactivité de groupements aldéhydes/cétones avec des groupements hydrazides/aminooxy.

Les groupes cétones et aldéhydes ont en effet été largement utilisés pour faire des modifications de biomolécules en les incorporant lors de synthèses (ou de biosynthèses) sous la forme de monomère (sucres, acides aminés, ...) modifiés. Ces groupes permettent alors une modification ultérieure en les faisant réagir avec des hydrazines ou des aminooxy, assemblage de deux parties d'inhibiteurs, marquage avec des agents contrastant (voir par exemple Mahal, L.K.et al. Science, 1997. 276(5315): p. 1125-1128).

Malheureusement, l'utilisation des groupes cétones et aldéhydes est limitée par le milieu biologique lui-même. En effet, les groupes carbonyles réagissent aussi avec les amines et ne sont pas spécifiques vis-à-vis des molécules biologiques en comportant. De plus, le pH optimal pour cette réaction de condensation est de 5-6 ; la réaction est donc soit très lente, soit l'acidification du milieu entraîne une certaine mort cellulaire ou la décomposition de biomolécules sensibles.

Un autre type de réaction de couplage ayant été considéré pour la chimie *in vivo* est une réaction qui repose sur l'interaction d'un azoture avec soit une phosphine (réaction de Staudinger) soit un alcyne (cycloaddition 1,3 dipolaire de Huisgen), (voir par exemple Agard, N.J., et al., Acs Chemical Biology, 2006. 1(10): p. 644-648).

Le groupement azoture s'avère avantageusement inerte à l'égard des groupes fonctionnels rencontrés dans le monde du vivant, très énergétique et peu réactif à l'égard d'un grand nombre de groupements.

Malheureusement, le frein majeur à l'utilisation du premier type d'interaction est la mise en oeuvre d'une phosphine facilement oxydable à l'air.

Quant aux cycloadditions de Huisgen avec des groupes azotures elles ne sont possibles qu'ex *vivo,* au regard du fait qu'elles requièrent l'emploi de sels de cuivre, exclus *in vivo* compte-tenu de leur toxicité.

Il est certes possible de s'affranchir de la présence du cuivre en activant les alcynes par des groupes électroattracteurs ; cependant, ces composés sont alors susceptibles de subir des réactions de Michael dans un milieu biologique riche en nucléophiles. Une autre alternative pour accélérer la cycloaddition 1,3 dipolaire des azotures sur les alcynes, consiste à faire la réaction sur des liaisons multiples contraintes, la contrainte de cycle étant la force motrice de la réaction (et remplaçant la catalyse). Le groupe de Bertozzi a ainsi tiré parti de l'accélération de la réaction due à la contrainte de cycle pour mettre au point une cycloaddition (3+2) entre azotures et cyclooctynes ayant lieu en conditions physiologiques et sans toxicité apparente, pour pouvoir procéder à des modifications chimiques sur les cellules dans un organisme vivant (voir par exemple Agard, et al, Journal of the American Chemical Society, 2004. 126(46): p. 15046-15047 et Bertozzi, C.R., et al., Compositions and methods for modification of biomolecules. 2006. p. 1-58).

Malheureusement, les azotures, en se révélant sensibles à la photo-dégradation et en étant pour certains d'entre eux potentiellement explosifs s'avèrent peu compatibles pour un usage en milieu biologique

Or, au regard de leur sélectivité, les cycloadditions 1,3-dipolaires, constituent manifestement une voie de fonctionnalisation dont il serait particulièrement avantageux de pouvoir tirer profit en milieu biologique.

Comme alternative aux cycloadditions 1,3-dipolaires azoture/alcène, il existe les cycloadditions 1,3-dipolaires nitrones/alcènes. Ces dernières permettent l'accès à des dérivés isoxazolidines, qui peuvent ensuite être convertis en de nombreux produits intéressants, notamment des β-lactames, des lactones, des aminoalcools et des cétoalcools. Cette réaction est très populaire et fournit deux paires de diastéréoisomères résultant des approches *endo* ou *exo.* Le même type de réaction entre nitrone et alcyne permet l'obtention d'isoxazolines, qui peuvent ensuite subir divers réarrangements, fournissant de nouveaux systèmes hétérocycliques.

Toutefois, ces cycloadditions 1,3-dipolaires de nitrones sur des insaturations sont généralement conduites d'une part en milieu organique et d'autre part sous températures élevées. Concernant la première contrainte, elle est généralement imposée par le fait que la condensation d'un aldéhyde et d'une hydroxylamine nécessite des conditions anhydres pour former la nitrone correspondante. Il a toutefois été montré que l'emploi d'un surfactant permet de former une nitrone et de procéder à la cycloaddition « one pot » de cette dernière avec l'acrylate d'éthyle (voir par exemple Chatterjee, A., et al, Organic Letters, 2003. 5(21): p. 3967-3969).

La présente invention repose plus particulièrement sur l'observation par les inventeurs que l'interaction d'une nitrone avec un groupement spécifique s'avère réalisable dans des conditions douces et donc compatibles avec une mise en oeuvre *in vivo,* c'est-à-dire en milieu aqueux et à une température pouvant varier de la température ambiante à la température corporelle.

Plus précisément, la présente invention se rapporte à un procédé utile pour greffer au moins une molécule d'intérêt à une entité annexe comprenant au moins les étapes consistant à
- disposer de ladite molécule pourvue, en surface d'au moins un groupement G1 réactif selon une réaction de cycloaddition 1,3-dipolaire, et
- mettre en présence ladite molécule avec ladite entité pourvue en surface d'au moins un groupement G2 complémentaire du groupement G1 au regard de ladite cycloaddition 1,3-dipolaire, dans des conditions propices à ladite cycloaddition desdits groupements G1 et G2,
caractérisé en ce que lesdits groupements G1 et G2 sont respectivement une nitrone et un radical cycloalcynyle contraint ou inversement.

Au sens de la présente invention, l'expression « lesdits groupements G1 et G2 sont respectivement une nitrone et un radical cycloalcynyle contraint ou inversement » signifie que le groupement G1 est une nitrone et le groupement G2 un radical cycloalcynyle contraint ou que le groupement G1 est un radical cycloalcynyle contraint et le groupement G2 une nitrone.

Le procédé selon l'invention est avantageusement compatible avec une mise en oeuvre *in vitro, ex viva* et/ou *in vivo.*

Ainsi, ladite cycloaddition est réalisable et de préférence est réalisée en un milieu assimilable à un milieu biologique et plus particulièrement en milieu aqueux.

Selon une autre variante préférée, ladite cycloaddition est réalisable et de préférence est réalisée à température ambiante ou à une température corporelle.

Selon encore une autre variante préférée, ladite cycloaddition est réalisée in vivo en surface d'un tissu ou au sein d'un organisme vivant.

Comme il ressort des exemples figurant çi-après, les inventeurs ont constaté que la réaction de cycloaddition 1,3-dipolaire entre une nitrone et un cycloalcyne contraint est apte à se réaliser dans des conditions douces et compatibles avec la biologie à savoir à température ambiante, en milieu aqueux, et ne requiert pas la mise en oeuvre d'additif annexe pour son activation.

Ainsi, le procédé selon l'invention permet notamment de s'affranchir de l'usage d'additifs, du type catalyseur, base, ou ligand (nécessaire dans la chimie clic classique) et de s'affranchir des problèmes de toxicité du cuivre(I) ou des ligands cryptants, des variations de pH physiologiques dommageables pour la viabilité cellulaire, et de simplifier les problèmes de biodisponibilité dans le cas d'une réaction menée *in vivo.*

De plus, les inventeurs ont constaté que l'interactivité d'un groupement nitrone pour un motif cycloalcyne contraint s'avère particulièrement avantageuse au regard de celle manifestée par un groupement azoture à l'égard d'un même motif cycloalcyne.

Tout d'abord, la réaction de cycloaddition entre nitrone et cycloalcyne contraint forme un cycloadduit qui se réarrange pour libérer une amine qui peut à son tour servir au couplage d'une autre molécule sur l'assemblage précédemment construit par la réaction de cycloaddition.

En outre, les composés comportant un groupement azoture sont potentiellement explosifs et par conséquent difficilement manipulables au contraire de ceux comportant un groupement nitrone.

Ainsi, le procédé selon l'invention permet, dans des conditions compatibles avec le vivant, de procéder au greffage d'une biomolécule d'intérêt à une autre entité en préservant les conformations des bio-polymères de façon générale, telles que les structures tertiaires et quaternaires des protéines, l'hybridation des brins d'ADN, des complexes ARN-ADN, des structures complexes ADN triples hélices ou quadruples hélices... qui sont stabilisées par des liaisons faibles, comme les liaisons hydrogène.

Au sens de la présente invention, l'entité annexe peut être une autre molécule d'intérêt ou un substrat.

Le procédé selon l'invention permet d'une part soit de coupler des biomolécules telles que par exemple les acides nucléiques, protéines, enzymes, anticorps, oligosaccharides, lipides,..., à d'autres molécules telles que par exemple des agents de contraste, molécules radiomarquées, vecteurs de transport, ligands, voire une autre biomolécule des types précédemment cités), soit de fonctionnaliser des substrats tels par exemple des nanoparticules organiques ou inorganiques, des biomatériaux, des tissus vivants, des nanocristaux, voire encore de modifier chimiquement une molécule afin de pallier par exemple sa sensibilité native à la température, aux solvants organiques, aux conditions basiques, et/ou à la présence de métaux de transition ou de cryptant.

Bien entendu, le procédé selon l'invention n'est aucunement limité à un usage à l'égard d'un environnement biologique. Il s'avère également particulièrement utile pour coupler deux molécules d'intérêt chimiques, greffer une molécule chimique sur une surface fonctionnalisée, greffer une biomolécule à la surface d'une nanoparticule voire modifier chimiquement des tissus vivants.

En conséquence, selon un autre de ses aspects, la présente invention concerne un procédé utile pour fonctionnaliser la surface d'un substrat avec au moins une molécule d'intérêt comprenant au moins les étapes consistant à :
- disposer de ladite molécule pourvue, en surface d'au moins un groupement G1 réactif selon une réaction de cycloaddition 1,3-dipolaire, et
- mettre en présence ladite molécule avec ledit substrat pourvue en surface d'au moins un groupement G2 complémentaire du groupement G1 au regard de ladite cycloaddition 1,3-dipolaire, dans des conditions propices à la cycloaddition desdits groupements G1 et G2,
caractérisé en ce que lesdits groupements G1 et G2 sont respectivement une nitrone et un radical cycloalcynyle contraint ou inversement.

Selon une variante de réalisation ledit substrat est un matériau dédié à un usage biologique.

Il peut notamment s'agir d'une puce à ADN, d'une cellule pour les tests de screening à haut-débit (activités enzymatiques, tests de cytotoxicité d'agents anticancéreux, antibactériens...).

Un tel procédé est également particulièrement utile pour la fonctionnalisation de polymères spécifiques et/ou de biopolymères, et pour la synthèse de structures de type dendritiques, d'hydrogels, de phospholipides, de lipides, de nanoparticules lipidiques telles que les liposomes, les nanocapsules, les SLN (Solid Lipide Nanoparticules) ou les nanoémulsions lipidiques.

Selon encore un autre de ses aspects, la présente invention concerne un procédé utile pour coupler deux molécules d'intérêt comprenant au moins les étapes consistant à :
- disposer d'une première molécule d'intérêt pourvue, en surface d'au moins un groupement G1 réactif selon une réaction de cycloaddition 1,3-dipolaire, et
- mettre en présence ladite molécule avec ladite seconde molécule d'intérêt pourvue en surface d'au moins un groupement G2 complémentaire du groupement G1 au regard de ladite cycloaddition 1,3-dipolaire, dans des conditions propices à la cycloaddition desdits groupements G1 et G2,
caractérisé en ce que lesdits groupements G1 et G2 sont respectivement une nitrone et un radical cycloalcynyle contraint ou inversement.

Un tel procédé est particulièrement utile pour la synthèse de molécules polyfonctionnalisées à visées thérapeutiques, pour le marquage de cellules ou de tissus vivants par des agents contrastants.

Ainsi, selon encore un autre de ses aspects, la présente invention concerne un procédé utile pour le marquage d'une molécule d'intérêt ou d'un substrat par un agent contrastant comprenant au moins les étapes consistant à :
- disposer de ladite molécule d'intérêt ou dudit substrat pourvu(e), en surface d'au moins un groupement G1 réactif selon une réaction de cycloaddition 1,3-dipolaire, et
- mettre en présence ladite molécule d'intérêt ou ledit substrat avec ledit agent contrastant pourvu en surface d'au moins un groupement G2 complémentaire du groupement G1 au regard de ladite cycloaddition 1,3-dipolaire, dans des conditions propices à la cycloaddition desdits groupements G1 et G2,
caractérisé en ce que lesdits groupements G1 et G2 sont respectivement une nitrone et un radical cycloalcynyle contraint ou inversement.

Un tel procédé est particulièrement utile pour le marquage de cellules ou tissus vivants.

Au sens de la présente invention, le « greffage » couvre les deux réactions qui suivent, à savoir :
- l'« ancrage » qui se réfère à l'immobilisation d'un composé, en l'occurrence une molécule d'intérêt en surface d'un substrat, et
- le terme « couplage » qui décrit la réaction entre au moins un groupe fonctionnel terminal d'un premier composé, notamment une première molécule d'intérêt, et au moins un groupe fonctionnel complémentaire, porté par une seconde molécule d'intérêt.

Selon une variante de réalisation, le procédé selon l'invention est mis en oeuvre à des fins d'ancrage de molécule(s) d'intérêt en surface d'un matériau de type substrat.

Ce substrat peut être par exemple, un support solide ou semi-solide.

Il peut être choisi parmi des surfaces de type métalliques, semi-conducteurs, oxydes, tels qu'alumines, SiO₂, TiO₂, organiques et biologiques.

Il peut s'agir en particulier d'un support convenant pour une utilisation en tant que puce à acides nucléiques (ADN, ARN, oligonucléotides), à protéine, à sucres, à cellules ou en tant que photosensibilisateur pour les cellules photovoltaïques, ou marqueur optique de billes de silice (pour les puces en suspension) ou encore d'un support biologique de type cellule ou tissus biologique.

Ces supports ou leurs surfaces à fonctionnaliser peuvent éventuellement être nanostructurés.

Ainsi, le support peut être constitué de particules, préférentiellement de diamètre compris entre 5 nm et 10 µm, et encore plus préférentiellement entre 5 nm et 300 nm. Ces particules peuvent être inorganiques ou organiques. Parmi les matériaux inorganiques, on peut citer des nanoparticules de type métalliques (par exemple : or, argent), semi-conducteurs (par exemple : silicium, CdSe, CdTe, CdSeTe, InP, InAs, PbS, CuInS₂), oxydes (par exemple : oxydes de fer, alumines, TiO₂, SiO₂). Parmi les nanoparticules de type organique, on peut citer les nanoparticules naturelles et synthétiques de polymères, de biopolymères, les structures de type dendritiques, les hydrogels, les nanoparticules lipidiques telles que les liposomes, les nanocapsules, les SLN (Solid Lipid Nanoparticles) ou les nanoémulsions.

Comme exemple de molécules d'intérêt, on peut notamment citer les marqueurs, les colorants, les fluorophores (tels que par exemple les coumarines, fluorescéine, fluorescines modifiées, rhodanines, iguanines, boron-dipynornéthène, oxazines et autres), les molécules radiomarquées, les agents de contraste (rayons X, IRM), les toxines (y compris les cytotoxines), les linkers, les actifs thérapeutiques, cosmétiques, phytosanitaires, les membres d'une paire liante spécifique, les peptides, les aminoacides et les résidus d'acides aminés, les polypeptides (y compris les peptides et les protéines), les sucres et les résidus de sucres, les photosensibilisateurs tels que par exemple l'éosine, le rose de bengal, les phtalocyanines, les chlorines, les bactériochlorines, les porphyrines telles que par exemple la tétrakis [;méso(4-éthynylphényl)porphyrine, la 1,1 0-(4-éthynylphényl)5,15- (4-mésityl)porphyrine et la 1-(4-éthynylphényl)5,10,15-(4-mésityl)porphyrine) dont la présence sur une surface est particulièrement utile dans le domaine de l'électronique moléculaire et de la photothérapie ; des composés présentant une isomérie cis-trans tels que les dérivés des diaryléthylènes, des spiropyrannes, des spiroxazines, des fulgides ou de l'azobenzène (dont la présence sur une surface est particulièrement utile pour la fabrication d'interrupteurs moléculaires photocontrôlés).

Selon une variante préférée, la molécule d'intérêt est une molécule biologique.

Le procédé de l'invention peut comprendre une mise en présence d'un groupe cycloalkynyle contraint présent au niveau du substrat, avec un groupe nitrone, présent au niveau de la molécule d'intérêt.

Toutefois, le procédé selon l'invention comprend généralement une mise en présence d'un groupement nitrone présent au niveau du substrat avec un groupe cycloalkynyle contraint, présent au niveau de la molécule d'intérêt.

Les biomolécules d'intérêt peuvent être d'origine naturelle, ou produites de manière synthétique ou recombinante, et peuvent être isolées, purifiées.

Lorsque la molécule d'intérêt est un polypeptide, le polypeptide peut être composé d'aminoacides D, L, ou des deux, et peut en outre être modifié, que ce soit de manière naturelle, synthétique, ou recombinante, afin d'y inclure d'autres groupements. Par exemple, le polypeptide cible peut être une lipoprotéine, une glycoprotéine, ou tout autre protéine modifiée.

Selon une autre variante de réalisation, le procédé selon l'invention est mis en oeuvre pour coupler au moins deux molécules d'intérêt notamment telles que définies ci-dessus.

Selon une autre variante de réalisation, la ou l'une des molécules d'intérêt est un agent contrastant.

### Cycloalcynyle contraint

Au sens de la présente invention, le terme « cycloalcynyle contraint » s'étend aux hétérocycloalcynyles pouvant comprendre, le cas échéant, un atome d'azote, d'oxygène, de soufre, de phosphore ou de silicium. Plus particulièrement, il s'agit d'un cycle à 8 ou 9 chaînons.

Selon un mode préféré de l'invention, le groupe cycloalcynyle est un groupe cyclooctynyle.

La contrainte appliquée sur le groupe cycloalcynyle peut être accrue de diverses manières, par exemple par l'utilisation d'hétéroatomes, le degré d'insaturation, ou la déformation due à la torsion, l'utilisation de groupes électroattracteurs, etc. Les dérivés correspondants sont également couverts sous le terme « cycloalcynyle contraint ».

Ainsi, le cycloalcynyle contraint conforme à l'invention peut être un composé dans lequel un ou plusieurs atome(s) de carbone du cycle cycloalcynyle, à l'exclusion des deux atomes de carbone joints par une triple liaison, est substitué par un ou plusieurs groupe(s) électroattracteur(s), par exemple un groupement halo (bromo, chloro, fluoro, iodo), nitro, un groupement cyano ou un groupement sulfone. Le groupe électroattracteur est placé de façon préférentielle en alpha de la triple liaison.

Comme autre groupe susceptible d'être présent sur le cycloalcynyle, on peut citer, de manière non exhaustive, les groupes carboxyle, amine, (par exemple alkyl amine (par exemple alkyl amine inférieur), aryl amine), ester (par exemple alkyl ester (par exemple alkyl ester inférieur, benzyl ester), aryl ester, aryl ester substitué), thioester, sulfonyl halide, alcool, thiol, ester succinimidyle, isothiocyanate, iodoacétamide, maléimide, hydrazine, etc.

La fonctionnalisation d'une molécule d'intérêt ou d'un substrat avec un tel groupement cycloalcynyle relève clairement des compétences de l'homme de l'art.

D'une manière générale, ce groupement cycloalcynyle est lié à de manière covalente à la molécule d'intérêt ou le substrat considéré via un bras espaceur.

Selon un mode de réalisation particulier ce bras espaceur peut être de formule comme suit

(I) -X-E-A-G

dans laquelle :
- X, lié à la molécule d'intérêt ou l'entité annexe considérée, est le produit réactionnel de deux fonctions réactives telles que, par exemple, thiol/maléimide, amine/acide carboxylique activé ou carbonyle/oxyamine,
- E est un groupe espaceur organique,
- A représente une liaison simple ou un groupe choisi parmi -CONH-, - NHCO-, OCH₂CONH-, -NHCOCH₂O-, -O-, -S-. et
- G est le groupement cycloalcynyle, contraint.

Le groupe E peut être un groupe hydrocarboné comprenant une ou plusieurs insaturations, par exemple, du type alcénique. Un exemple d'un tel groupe peut être un groupe alkylène tel que défini ci-dessus interrompu par une ou plusieurs insaturations alcéniques. Lorsque le groupe E comporte au moins deux insaturations, il peut conférer aux composés, une capacité à se réticuler.

Le groupe E peut être également une chaîne poly(ethylène) glycol (PEG). Un tel groupe permet d'améliorer la solubilité de la molécule d'intérêt ou du substrat considéré, voire celle du produit de couplage attendu.

Cette variante de réalisation de E est particulièrement avantageuse pour la fonctionnalisation de matériaux de type polymère, dendrimères, oligomères, particules lipidiques, hydrogels apte par exemple à encapsuler des nanocristaux semi-conducteurs fluorescent, des ftuoroplaores ou tout autre agent d'intérêt diagnostique ou thérapeutique dans des matériaux, gels, ou formulation (matériaux plastiques pour l'optique et l'électronique, délivrance d'agents de photothérapie, par exemple), ou encore à servir de matrice- support pour la reconstruction tissulaire.

Le groupe E peut être également un groupe hydrocarboné comprenant un ou plusieurs groupes aromatiques. On peut citer, par exemple, un groupe comprenant des groupes aromatiques conjugués avec des groupes linéaires insaturés, tel qu'un groupe résultant de l'enchaînement d'un motif phénylène-vinylène. Ces groupes contribuent à conférer des propriétés optiques non linéaires.

Le groupe E peut également être une petite molécule organique permettant d'apporter la fonction réactive complémentaire, par exemple l'éthylène diamine, l'acide glycolique, la cystéamine, ou tout autre molécule organique permettant d'apporter des acides/amines/thiols.

On peut également citer un groupe comprenant des motifs pyrrole et/ou thiophène. Ces groupes contribuent à conférer au matériau des propriétés des conductions électroniques. On peut citer, par exemple, un groupe comprenant un ou plusieurs aromatiques substitués par un ou plusieurs groupes aromatiques, tel qu'un groupe comprenant un enchaînement de motifs quinones ou de motifs diazoïques. Ces groupes contribuent à conférer au composé, les possédant, des propriétés de photo/électroluminescence.

### Nitrone

La synthèse de groupements nitrone relève des compétences de l'homme de l'art.

Par exemple, une des réactions classiquement considérées pour former un tel groupement est la condensation d'une hydroxylamine N-monosubstituée avec soit une cétone soit une aldéhyde.

Dans l'hypothèse, où un tel groupement ne peut être généré directement sur l'une des molécules d'intérêt considérées dans le procédé de l'invention, celui-ci peut être crée en surface de ladite molécule via l'interaction d'une fonction réactive de ladite molécule d'intérêt avec une fonction réactive complémentaire figurant sur entité annexe portant ledit groupement nitrone.

Ainsi, selon une variante particulière de l'invention, le groupement nitrone peut être lié à ladite molécule d'intérêt via un bras espaceur organique E tel que défini précédemment et, de préférence, de nature hydrocarbonée en C₃ à C₁₀ ou une chaîne polyéthylène glycol.

Comme précisé précédemment, la réaction de cycloaddition est avantageusement obtenue sans catalyseur ou autres additif.

L'énergie d'activation nécessaire à la réaction est fournie par le groupement nitrone et le groupement cycloalcynyle contraint.

La cylcoaddition peut être réalisée avantageusement en milieu aqueux et à température ambiante ou corporelle, soit une température variant de 16 à 45 °C.

Au sens de l'invention, un milieu aqueux désigne de l'eau pure ou un mélange majoritairement formé d'eau et contenant en outre un ou plusieurs solvants annexes. De tels mélanges sont assimilables à de l'eau pure.

Au sens de l'invention, la température ambiante peut varier de 16 à 28 °C et de préférence de 18 à 25 °C.

Au sens de l'invention, on entend par température corporelle, des températures comprises entre 32 et 45 °C, plus particulièrement entre 36 et 42 °C, et essentiellement proche de 37 à 38 °C.

Ainsi, le procédé selon l'invention peut être réalisé avantageusement *in vivo* dans un tissu ou un organisme vivant.

Les procédés selon l'invention ont de multiples applications, que ce soit en synthèse, recherche ou en diagnostique.

Les applications de recherche comprennent également la découverte d'un médicament ou des applications de dépistage/criblage : comme tout agent de contraste, l'invention permet la visualisation de molécules d'intérêt (récepteur, glucose, antigène ...) ou de mécanismes biologiques (endocytose, néoangiogenèse, apoptose, activités enzymatiques ...) et permet donc l'identification de cibles thérapeutiques.

Elle permet aussi de faire un suivi de l'effet d'un traitement médicamenteux (par exemple cytotoxique, inhibiteur d'une enzyme ...) ou la détection de tumeurs (par l'intermédiaire de marqueurs spécifiques).

Comme autres applications d'intérêt, on peut citer l'étude des caractéristiques fonctionnelles et physiques d'un récepteur, la protéomique, la métabolomique, etc.

L'invention peut également être utilisée pour le développement de matrices pour la reconstruction tissulaire (hydrogels, biomatériaux synthétiques ou modification de surface de matériaux implantables), et d'une façon générale pour le développement de dispositifs médicaux.

L'invention peut également être utilisée pour la fabrication de nanoparticules fonctionnalisées (nanoparticules organiques ou inorganiques). Ces nanoparticules peuvent être employées par exemple pour la délivrance de principes actifs thérapeutiques, comme outils de diagnostique médical ou pour le guidage du geste chirurgical. Comme outil de diagnostic, on peut citer en particulier leur utilisation en imagerie moléculaire.

Pour certaines applications dans le domaine médical ou préclinique, comme par exemple l'imagerie moléculaire, la réaction de couplage entre l'entité porteuse du groupement nitrone et celle porteuse du groupement cycloalcyne contraint peut être réalisée ex vivo ou in vivo après injection simultanée ou différée dans le temps des deux entités.

Les exemples et figures qui suivent sont présentés à titre illustratif et non limitatif au domaine de l'invention.
Figure 1 : Spectre XPS global des substrats fonctionnalisés par des nitrones.
Figure 2 : Spectre MIR-FTIR des lames fonctionnalisées par des nitrones.
Figure 3 : Analyse du spotting des sondes fluorescentes sur les lames fonctionnalisées par des nitrones, moyennes sur 5 lames.
Figure 4 Spectre XPS global après fonctionnalisation des lames nitrones par cycloaddition avec un cycloalcyne contraint.

### Exemple 1 :

### Cycloaddition entre une nitrone et un dérivé cycloalcyne contrant en milieu non biologique.

### 1. Synthèse de la N-propylidén-méthylnitrone (4)

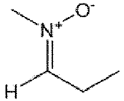

A un mélange de *N*-méthylhydroxylamine (835 mg ; 10 mmol) dans l'éthanol (15 mL) et de NaOMe (540 mg ; 10 mmol) sont ajoutés 725 µL de propionaldéhyde **2** (10 mmol). Le tout est agité 1 h à Tₐ. La solution est évaporée à sec, re-suspendue dans CH₂Cl₂, filtrée, et le filtrat purifié par chromatographie sur gel de silice (CH₂Cl₂/MeOH 0-10 %) pour obtenir la nitrone **4** sous forme de solide jaune de masse 500 mg. Le rendement est de 57 %.
RMN ¹H (CDCl₃, 200 MHz): δ(ppm 1.02 (t, J = 7.5 Hz, 3H, H₃) ; 2.40 (dqd, J = 1.0-7.5-6.0, 2H, H₂) ; 3.59 (d, J = 1.0, 3H, H₄) ; 6.62 (t, J = 6.0, 1H, H₁) ;

### 2.Synthèse de l'acide cyclooct-1-yn-3-glycolique (18)

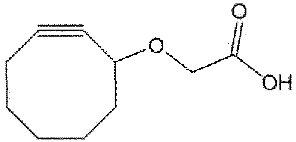

### a)Synthèse du 8,8-dibromobicyclo[5.1.0]cyclooctane (16)

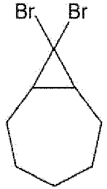

Dans un ballon sec et sous argon sont introduits 3,65 g de cycloheptène **15** (38 mmol) puis 8,52 g de *t*-BuOK (76 mol) et 9 mL de pentane anhydre. La solution, placée dans un bain glace/sel (T < -5°C), est agitée vigoureusement puis 4,9 mL de bromoforme (57 mmol) sont ajoutés au goutte à goutte. Une fois l'addition terminée, le mélange est laissé revenir à Tₐ toute la nuit, sous argon et agitation vigoureuse. Environ 50 mL d'eau sont ensuite ajoutés et le pH neutralisé avec HCl 1M. Les phases organique et aqueuse sont séparées ; la phase aqueuse est extraite avec 3x20 mL de pentane et la phase pentane est lavée avec 3x20 mL d'eau. Après séchage sur MgSO₄, le solvant est évaporé sous vide pour donner une huile jaune-orangée. Le produit **16** est ensuite purifié par filtration sur silice avec comme éluant un mélange cyclohexane/AcOEt 5 %. Une huile incolore de masse totale 9,10 g est alors obtenue, équivalent à un rendement de 90 % (litt. 52-65 % pour le 9,9-dibromobicyclo[6.1.0]nonane). R_{f} (cyclohexane 95/ AcOEt 5) = 0,85 ;
RMN ¹H (CDCl₃, 400 MHz): δ (ppm) 1.05-1.22 (m, 3H, H₄₋₂₋₆); 1.34 (qq, J = 1.0-7.5 Hz, 2H, H₃₋₅); 1.68 (ddd, J = 1.5-4.0-10.5 Hz, 2H, H₁₋₇) ; 1.76-1.92 (m, 3H, H_{4'-3'-5'}) ; 2.23 (dtq, J = 14.0-6.0-1.0 Hz, 2H, H_{2'-6'}) ;

### b) Synthèse du 2-bromocyclooct-1-èn-3-glycolate de méthyle (17)

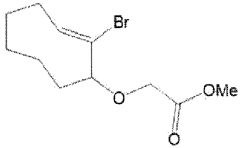

A une solution de 8,8-dibromobicyclo[5.1.0]octane **16** (2,5 g; 9,3 mmol) et de glycolate de méthyle (6,35 mL ; 83,9 mmol) dissous dans 5 mL de toluène anhydre dans un ballon sec, sous Ar et protégé de la lumière par un film d'aluminium, sont ajoutés 3,85 g de perchlorate d'argent (18,6 mmol). La réaction est agitée pendant 1 h 30 à température ambiante, puis les sels d'argent sont filtrés sur fritté et lavés avec AcOEt. La solution est concentrée sous vide pour donner une huile visqueuse brune qui est purifiée par chromatographie sur gel de silice (cyclohexane/AcOEt 2-15 %), pour obtenir **17** sous forme d'huile jaune (m = 1,7 g, soit 66 % de rendement).
RMN ¹H (CDCl₃, 200 MHz) : δ (ppm) 0.7-2.2 (m, 8H, H_{4-4'-5-5'-6-6'-7-7'}) ; 2.28 (m, 1H, H₈) 2.70 (ddd, J = 5.0-11.5-23.5 Hz, 1H, H_{8'}) ; 3.72 (s, 3H, OMe) ; 3.94 (d, J = 16.5 Hz, 1H, H₉) ; 4.10 (dd, J = 5.0-10.0 Hz, 1H, H₃) ; 4.23 (d, J = 16.5 Hz, 1H, H_{9'}) ; 6.20 (dd, J = 4.0-11,5 Hz, 1H, H₁) ;

### b) Synthèse de l'acide cyclooct-1-yn-3-glycolique (18)

A une solution de 2-bromo-cyclooctèn-3-glycolate de méthyle **17** (0,5 mmol) dans le DMSO anhydre à 60°C, sont ajouté 150µL de 1,8-diazabicyclo[5.4.0]undec-7-ène (DBU, 1 mmol). Après 15 min d'agitation, 600 µL supplémentaires de DBU (4 mmol) sont ajoutés, puis le tout est agité à 60°C pendant une nuit. Le mélange est acidifié avec HC1 1M et extrait avec AcOEt. La phase organique est filtrée sur filtre séparateur et est concentrée sous vide. L'acide cyclooct-1-yn-3-glycolique **18** est purifié sur plaque préparative (CH₂Cl₂/MeOH 9/1) et est obtenu avec un rendement de 55 % (m = 50 mg).
RMN ¹H (CDC13, 200 MHz): δ(ppm 1.3-2.3 (m, 10H, cycle); 4.45 (d, J = 17.0 Hz, 1H, H₉) ; 4.50 (m, 1H, H₃) ; 4.58 (d, J = 17.0 Hz, 1H, H_{9'}) ; 8.12 (s, 1H, H₁₁) ;

### 3.Cycloaddition

A une solution de **18** (70 mg ; 0,38 mmol) dans un mélange eau/THF (1 ml/200µl) est ajoutée la nitrone **4** (33 mg ; 0,38 mmol). Le tout est agité à Tₐ pendant 2 jours. Puis le produit est extrait avec CH₂Cl₂ et concentré sous-vide. Le produit attendu étant trop polaire pour être purifié sur gel de silice et les analyses sont réalisées sur le brut de la réaction qui révèle un produit très majoritaire.

Le rendement de la réaction apprécié par mesure du taux de conversion en RMN est d'environ 85% (calculé à partir du pic résiduel du CH_{Nit}). 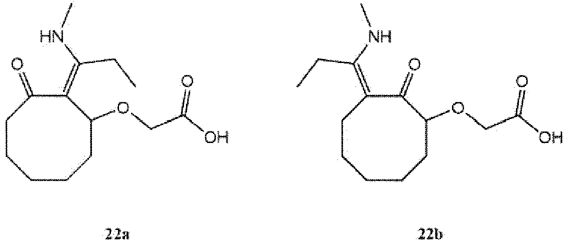 RMN ¹H (CDCl₃, 200 MHz) 22a ou b : δ(ppm) 0.8-2.3 (m, 13H, cycle + H₁₃) ; 2.3-2.6 (m, 2H, H₁₂₋₈) ; 2.8-2.8 (m, 1H, H_{12'}) ; 3.6-3.8 (m, 1H, H₃) ; 3.8-4.3 (m, 2H, H_{9/9'}) ; 8.30 (s, 1H, NH) ;
RMN ¹³C (CDCl₃, 200 MHz) 22a ou b_: δ(ppm 9.60 (C₁₃) ;19.81 (C₁₂); 20.51 ; 22.57 ; 27.95 ; 31.70 (C₅₋₈) ; 44.27 (C₄) ; 44.36 (C₁₄) ; 66.42 (C₃) ; 79.48 (C₉) ; 133.72 (C2) ; 135.83 (C₁₁) ; 173.26 (C₁₀) ; 213.69 (C₁) ;
Masse ESI⁺ m/z [M-CH₂CO₂H+H]⁺ = 212.4 pour 212.2 ;

### Exemple 2 :

### Préparation de surfaces fonctionnalisées par des groupes nitrones et greffage d'une sonde fluorescente par réaction de cycloaddition avec un cycloalcyne contraint

### 1. Préparation des lames de verre fonctionnalisées par des nitrones

Les surfaces fonctionnalisées par des nitrones sont obtenues *via* les 4 étapes suivantes :

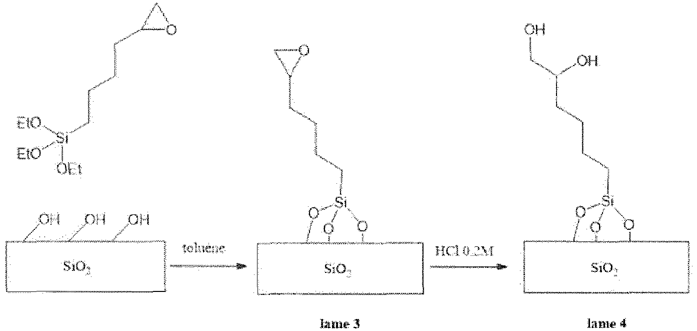

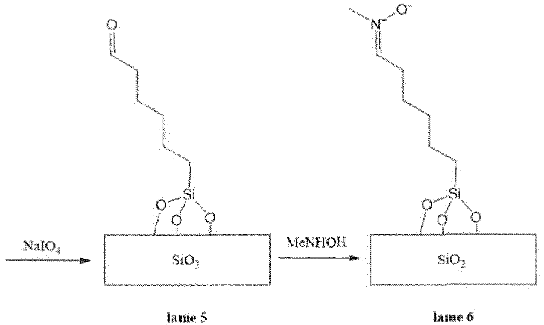

Les lames de verre sont tout d'abord activées par une solution de Brown pour hydroxyler la surface. Puis la silanisation est réalisée dans le toluène en présence de 5,6-époxyhexyltriéthoxysilane à température ambiante et en présence de diéthylamine pour donner les lames **3.** L'ouverture de l'époxyde en diol 1,2 est réalisée en milieu acide, puis l'aldéhyde est obtenu par oxydation par le periodate de sodium à la surface des lames **4.**

La dernière étape consiste à la condensation de la *N-*méthylhydroxylamine sur l'aldéhyde des lames **5** dans l'éthanol, en présence de méthylate de sodium et à température ambiante.

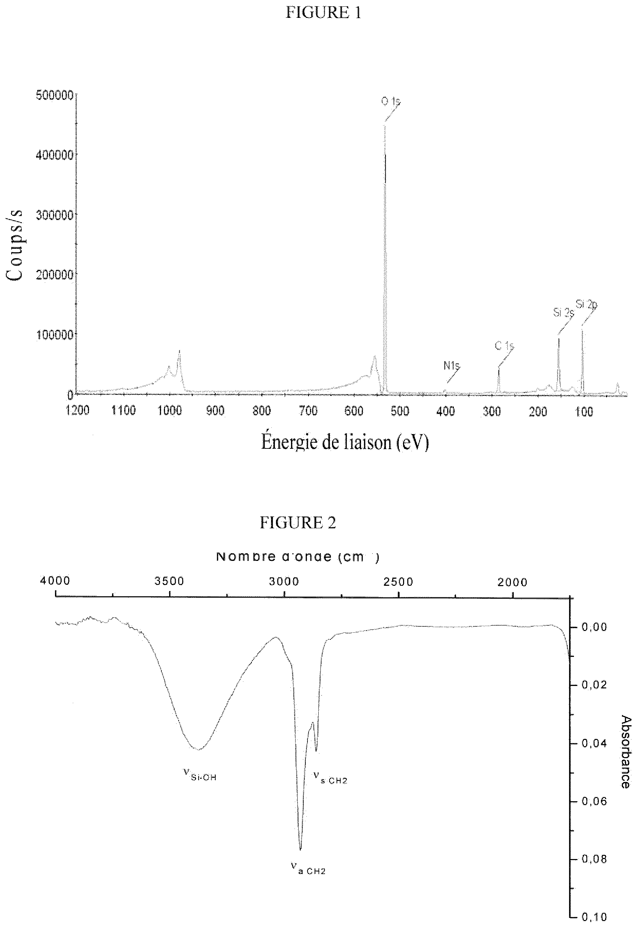

Les lames **6** ainsi obtenues ont été caractérisés par leurs spectres XPS (figure 1) et MIR-FTIR (figure 2).

Les signaux correspondants à Si (103 et 154 eV), à C (286 eV), à O (533 eV) et à N (399 eV) sont présents sur le spectre XPS des lames **6.** La présence de l'ensemble de ces signaux atteste de la fonctionnalisation réussie de la surface des lames **6.**

Par spectre MIR-FTIR, la fonctionnalisation de la lame est également visible à la présence des bandes de vibration d'élongation des CH₂ à 2931 et 2860 cm⁻¹.

### 2. Synthèse de l'Alexa-cyclooctyne

A une solution d'acide cyclooct-1-yn-3-glycolique **18** (10 mg ; 55 µmol) dans 250 µl d'un mélange DMF/eau (8/2) sont ajoutés 1 µL de NEt₃ (7,4 µmol), 16 mg d'EDC(1-éthyl-3-(3-diméthylaminopropyl)carbodiimide) (83 µmol), 10 mg d'HOBt (N-hydroxybenzotriazole) (74 µmol) et 1 mg d'Alexa 488 cadaverine **26** (1,5 µmol) (commercialisée par INVITROGEN). La réaction est agitée une nuit à Tₐ puis est purifiée par HPLC (Annexe E). La sonde Alexa-cyclooctyne **27** est obtenue avec un rendement évalué à 25 % par quantification UV à partir de la loi de Beer-Lambert. 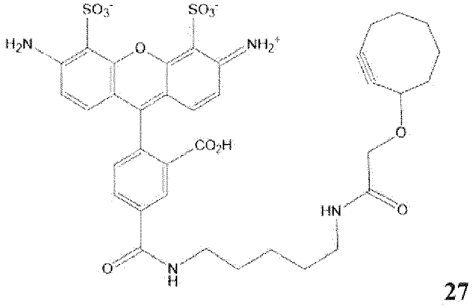 Masse ESI⁻ m/z : [M-Na]- = 781.3 pour 781.2 ;

### 3. Greffage par cycloaddition entre nitrone de surface et sonde fluorescente portant un cycloalcyne contraint

Il a été testé 6 concentrations différentes en sondes fluorescentes. (en présence de 10 % de glycérol, afin de faciliter la lecture des résultats)

Les concentrations choisies sont 50-100-250-500-750-1000 nM. Le plan de dépôt est représenté schématiquement par le tableau 1 ci-après.

**TABLEAU 1**

| Sonde **26** (témoin en sixplicat) | Sonde **27** (témoin en sixplicat) |
|---|---|
| 50 nM | 50 nM |
| 100 nM | 100 nM |
| 250 nM | 250 nM |
| 500 nM | 500 nM |
| 750 nM | 750 nM |
| 1 µM | 1 µM |

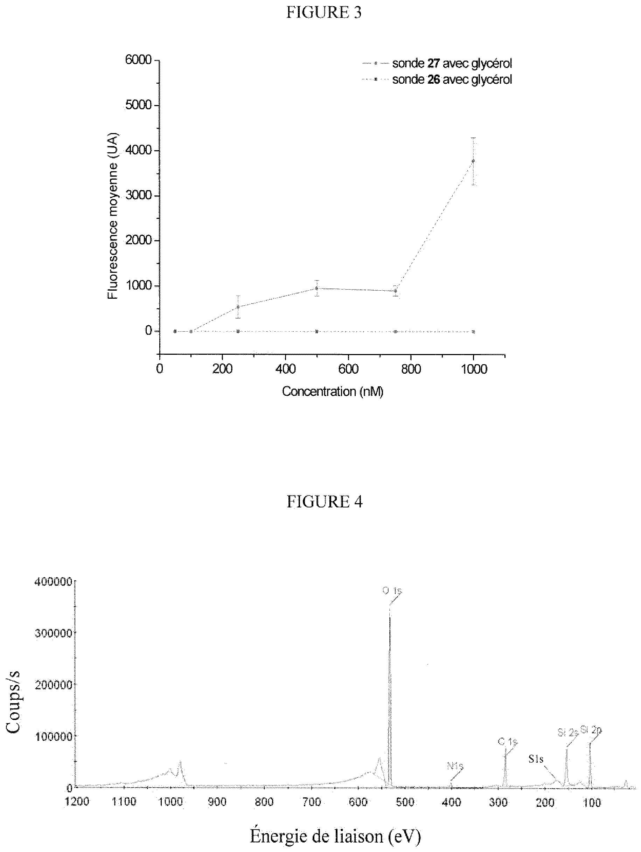

Le dépôt de gouttes de sondes fluorescentes a été mené sur les lames 6 (fonctionnalisées en surface par des groupes nitrone). Les résultats sont présentés en figure 3.

L'intensité moyenne de fluorescence est dépendante de la concentration en sonde fluorescente.

Pour vérifier que le signal observé avec la sonde Alexa-cyclooctyne **27** sur les lames est bien dû à la fonctionnalisation de la surface par chimie entre cycloalcyne contraint et nitrone, il a été procédé à la caractérisation de substrats Si/SiO₂ fonctionnalisés avec le groupe nitrone et incubés une nuit dans une solution de la sonde Alexa-cyclooctyne 27 à une concentration de 250 nM par mesure de l'angle de contact et spectre XPS.

Les angles de contact mesurés sur des substrats Si/SiO₂ fonctionnalisés de manière identique (portant des nitrones) avant et après fonctionnalisation par chimie(s) sur alcyne contraint sont résumés dans le tableau 2.

**TABLEAU 2**

| Substrats | *θᵢₙᵢₜᵢₐₗ* | *θ_{final}* |
|---|---|---|
| Substrat nitrone (type lame 6) | 47.3 ° | 53.5 ° |

Pour les surfaces de type lame 6, relativement hydrophiles, le greffage d'un fluorophore possédant de multiples noyaux aromatiques hydrophobes rend la surface des lames plus hydrophobes.

De la même façon, la modification des lames 6 par incubation avec la sonde Alexa-cyclooctyne **27** est visible par analyse XPS.

La figure 4 rend compte de cette analyse.

Le signal de l'azote reste globalement de même forme que précédemment mais avec une augmentation du signal à 399.6 eV, qui rend compte du greffage de la sonde Alexa-cyclooctyne **27** (comportant plusieurs azotes).

De plus, comme montré dans le tableau 3 ci-après, les rapports atomiques N/Si₂ₚ et C/Si₂ₚ augmentent après l'incubation avec la sonde Alexa-cyclooctyne **27,** indiquant la réussite du greffage.

**TABLEAU 3**

| Rapports atomiques | Avant incubation | Après incubation |
|---|---|---|
| N/Si₂ₚ | 0.043 | 0.078 |
| C/Si₂ₚ | 0.396 | 0.804 |

L'ensemble de ces analyses de la surface des lames après incubation avec la sonde Alexa-cyclooctyne 27 permet de conclure à une fonctionnalisation de la surface des lames 6 par réaction de cycloaddition [3+2] entre nitrones et cycloalcynes contraints.

## Revendications

1. Procédé utile pour greffer au moins une molécule d'intérêt à une entité annexe comprenant au moins les étapes consistant à
- disposer de ladite molécule pourvue en surface d'au moins un groupement G1 réactif selon une réaction de cycloaddition 1,3-bipolaire, et
- mettre en présence ladite molécule avec ladite entité pourvue en surface d'au moins un groupement G2 complémentaire du groupement G1 au regard de ladite cycloaddition 1,3-dipolaire, dans des conditions propices à la cycloaddition desdits groupements G1 et G2,
**caractérisé en ce que** lesdits groupements G1 et G2 sont respectivement une nitrone et un radical cycloalcynyle contraint ou inversement.

2. Procédé selon la revendication précédente, dans lequel la cycloaddition est réalisée en milieu aqueux.

3. Procédé selon l'une quelconque des revendications précédentes, dans lequel la cycloaddition est réalisée à une température ambiante ou corporelle.

4. Procédé selon l'une quelconque des revendications précédentes dans lequel ladite molécule d'intérêt est choisie parmi les marqueurs, les colorants, les fluorophores, les molécules radiomarquées, les agents de contraste (rayons X, IRM), les linkers, les toxines, les actifs thérapeutiques, cosmétiques, phytosanitaires, les membres d'une paire liante spécifique, les peptides, les aminoacides et les résidus d'aminoacide, les polypeptides, les sucres et les résidus de sucres, les photosensibilisateurs, les porphyrines des composés présentant une isomérie cis-trans tels que les dérivés des diaryléthylènes, des spiropyrannes, des spiroxazines, des fulgides et l'azobenzène.

5. Procédé selon l'une quelconque des revendications précédentes dans lequel ladite molécule d'intérêt est biologique.

6. Procédé selon l'une quelconque des revendications précédentes dans lequel l'entité annexe est également une molécule d'intérêt.

7. Procédé selon la revendication précédente dans lequel l'une des molécules d'intérêt est un agent contrastant.

8. Procédé selon l'une quelconque des revendications 1 à 5 dans lequel l'entité annexe est un substrat.

9. Procédé selon la revendication précédente, dans lequel le substrat est choisi parmi des surfaces de type métalliques, semi-conducteurs, oxydes, tels qu'alumines, SiO₂, TriO₂, organiques et biologiques.

10. Procédé selon la revendication précédente, dans lequel la surface est nanostructurée.

11. Procédé selon l'une quelconque des revendications précédentes dans lequel le radical cycloalcynyle est un radical cyclooctynyle.

12. Procédé selon l'une quelconque des revendications précédentes dans lequel le groupement cycloalcynyle contraint est lié à de manière covalente à la molécule d'intérêt ou à l'entité annexe via un bras espaceur.

13. Procédé selon la revendication précédente, dans lequel le bras espaceur est de formule
(I) -X-E-A-G
dans laquelle :
- X, lié à la molécule d'intérêt ou l'entité annexe considérée, est le produit réactionnel de deux fonctions réactives,
- E est un groupe espaceur organique,
- A représente une liaison simple ou un groupe choisi parmi -CONH-, -NHCO-,-OCH₂CONH-, -NHCOCH₂O-, -O-, -S-. et
- G est le groupement cycloalcynyle contraint.

14. Procédé selon la revendication précédente, dans lequel E est une chaîne poly(éthylène)glycol.

15. Procédé selon la revendication 1 utile pour le marquage d'une molécule d'intérêt ou d'un substrat par un agent contrastant comprenant au moins les étapes consistant à :
- disposer de ladite molécule d'intérêt ou dudit substrat pourvu(e), en surface d'au moins un groupement G1 réactif selon une réaction de cycloaddition 1,3-dipolaire, et
- mettre en présence ladite molécule d'intérêt ou dudit substrat avec ledit agent contrastant pourvu en surface d'au moins un groupement G2 complémentaire du groupement G1 au regard de ladite cycloaddition 1,3-dipolaire, dans des conditions propices à la cycloaddition desdits groupements G1 et G2,
lesdits groupements G1 et G2 étant respectivement une nitrone et un radical cycloalcynyle, contraint ou inversement.

## Patentansprüche

1. Verfahren zum Pfropfen wenigstens eines Zielmoleküls mit einer angehängten Einheit, umfassend zumindest die Schritte, die bestehen in:
- Bereitstellen des genannten Moleküls, das an der Oberfläche mit wenigstens einer reaktiven Gruppe G1 gemäß einer 1,3-dipolaren Zykloadditionsreaktion versehen ist und
- Konfrontieren des genannten Moleküls mit der genannten Einheit, die an der Oberfläche mit wenigstens einer Gruppe G2 versehen ist, die zu der Gruppe G1 hinsichtlich der genannten 1,3-dipolaren Zykloadditionsreaktion komplementär ist, unter Bedingungen, die für die Zykloaddition dieser Gruppen G1 und G2 geeignet sind,
**dadurch gekennzeichnet, dass** die Gruppe G1 ein Nitron und die Gruppe G2 ein gezwungenes Zykloalkinylradikal ist oder umgekehrt.

2. Verfahren nach dem vorstehenden Anspruch, bei dem die Zykloaddition in einem wässrigen Milieu erfolgt.

3. Verfahren nach einem der vorstehenden Ansprüche, bei dem die Zykloaddition bei Umgebungstemperatur oder Körpertemperatur erfolgt.

4. Verfahren nach einem der vorstehenden Ansprüche, bei dem das genannte Zielmolekül ausgewählt ist unter Markern, Färbemitteln, Fluorophoren, radioaktiv markierten Molekülen, Kontrastmitteln (Röntgen, MRT), Vemetzungsmitteln. Toxinen, therapeutisch aktiven Mitteln, Kosmetika, phytosanitären Mitteln, Gliedern eines spezifischen Bindungspaares, Peptiden, Aminosäuren und Aminosäureresten, Polypeptiden, Zuckern und Zuckerresten, Photosensibilatoren, Porphyrinen und Verbindungen, die eine Cis-Trans-Isomerie aufweisen, wie etwa Derivate von in Diarylethylenen, Spiropyranen, Spirooxazinen, Fulgiden und Azobenzenen.

5. Verfahren nach einem der vorstehenden Ansprüche, bei dem das genannte Zielmolekül biologisch ist.

6. Verfahren nach einem der vorstehenden Ansprüche, bei dem die genannte angehängte Einheit ebenfalls ein Zielmolekül ist.

7. Verfahren nach dem vorstehenden Anspruch, bei dem das eine der Zielmoleküle ein Kontrastmittel ist.

8. Verfahren nach einem der Ansprüche 1 bis 5, bei dem die angehängte Einheit ein Substrat ist.

9. Verfahren nach dem vorstehenden Anspruch, bei dem das Substrat ausgewählt ist unter Oberflächen vom Typ metallischer Oberflächen, von Halbleitern, Oxiden wie etwa Aluminiumoxiden, SiO₂, TiO₂, organischen und biologischen Substanzen.

10. Verfahren nach dem vorstehenden Anspruch, bei dem die Oberfläche nanostrukturiert ist.

11. Verfahren nach einem der vorstehenden Ansprüche, bei dem das Zykloalkinylradial ein Zyklooctinylradikal ist.

12. Verfahren nach einem der vorstehenden Ansprüche, bei dem die gezwungene Zykloalkinylgruppe über einen Abstandshaltearm kovalent an das Zielmolekül oder die angehängte Einheit gebunden ist.

13. Verfahren nach dem vorstehenden Anspruch, bei dem der Abstandshaltearm die folgende Formel hat
(I) -X-E-A-G
wobei
- X, gebunden an das Zielmolekül oder die betreffende angehängte Einheit, das Reaktionsprodukt von zwei reaktiven Funktionen ist,
- E eine organische Abstandshaltergruppe ist,
- A eine Einfachbindung oder eine Gruppe repräsentiert, die ausgewählt ist unter -CONH-, -NHCO-, -OCH₂CONH, -NHCOCH₂O-, -O-, -S-, und
- G die gezwungene Zykloalkinylgruppe ist.

14. Verfahren nach dem vorstehenden Anspruch, bei dem E eine Poly(ethy)glykolkette ist.

15. Verfahren nach Anspruch 1, zum Markieren eines Zielmoleküls oder eines Substrats mit einem Kontrastmittel, das wenigstens die Schritte aufweist, die bestehen in:
- Bereitstellen des genannten Zielmoleküls oder des genannten Substrats, das oder die an der Oberfläche mit wenigstens einer reaktiven Gruppe G1 gemäß einer 1,3 bipolaren Zykloadditionsreaktion versehen ist (sind), und
- Konfrontieren des genannten Zielmoleküls oder des genannten Substrats mit dem genannten Kontrastmittel, das an seiner Oberfläche mit wenigstens einer Gruppe G2 versehen ist, die im Hinblick auf die genannte 1,3-dipolare Zykloadditionsreaktion zu der Gruppe G1 komplementär ist, unter Bedingungen, die für die Zykloaddition der genannten Gruppen G1 und G2 geeignet sind,
wobei die Gruppe G1 ein Nitron und die Gruppe G2 ein gezwungenes Zykloalkinylradikal ist oder umgekehrt.

## Claims

1. Process of use in grafting at least one molecule of interest to an additional entity comprising at least the stages consisting in
- having available said molecule provided, at the surface, with at least one group G1 which is reactive according to a 1,3-bipolar cycloaddition reaction, and
- bringing together said molecule and said entity provided, at the surface, with at least one group G2 complementary to the group G1 from the viewpoint of said 1,3-dipolar cycloaddition, under conditions favorable to the cycloaddition of said groups G1 and G2,
**characterized in that** said groups G1 and G2 are respectively a nitrone and a strained cycloalkynyl radical or vice versa.

2. Process according to the preceding claim, in which the cycloaddition is carried out in an aqueous medium.

3. Process according to any one of the preceding claims, in which the cycloaddition is carried out at ambient temperature or at body temperature.

4. Process according to any one of the preceding claims, in which said molecule of interest is chosen from labels, colorants, fluorophores, radiolabeled molecules, contrast agents (X-ray, MRI), linkers, toxins, therapeutic, cosmetic or plant-protection active principles, members of a specific binding pair, peptides, amino acids and amino acid residues, polypeptides, sugars and sugar residues, photosensitizers, porphyrins compounds exhibiting cis-trans isomerism, such as diarylethylene derivatives, spiropyrans, spiroxazines, fulgides and azobenzene.

5. Process according to any one of the preceding claims, in which said molecule of interest is biological.

6. Process according to any one of the preceding claims, in which the additional entity is also a molecule of interest.

7. Process according to the preceding claim, in which one of the molecules of interest is a contrast agent.

8. Process according to any one of Claims 1 to 5, in which the additional entity is a substrate.

9. Process according to the preceding claim, in which the substrate is chosen from surfaces of metal, semiconductor, oxide, such as aluminas, SiO₂, TiO₂, organic and biological type.

10. Process according to the preceding claim, in which the surface is nanostructured.

11. Process according to any one of the preceding claims, in which the cycloalkynyl radical is a cyclooctynyl radical.

12. Process according to any one of the preceding claims, in which the strained cycloalkynyl group is covalently bonded via a spacer arm to the molecule of interest or to the additional entity.

13. Process according to the preceding claim, in which the spacer arm has the formula
(I) -X-E-A-G
in which:
- X, bonded to the molecule of interest or the additional entity under consideration, is the reaction product of two reactive functional groups,
- E is an organic spacer group,
- A represents a single bond or a group chosen from -CONH-, -NHCO-, -OCH₂CONH-, -NHCOCH₂O-, -O- or -S-, and
- G is the strained cycloalkynyl group.

14. Process according to the preceding claim, in which E is a poly(ethylene) glycol chain.

15. Process according to Claim 1 of use in the labeling of a molecule of interest or of a substrate by a contrast agent comprising at least the stages consisting in:
- having available said molecule of interest or said substrate provided, at the surface, with at least one group G1 which is reactive according to a 1,3-dipolar cycloaddition reaction, and
- bringing together said molecule of interest or said substrate and said contrast agent provided, at the surface, with at least one group G2 complementary to the group G1 from the viewpoint of said 1,3-dipolar cycloaddition, under conditions favorable to the cycloaddition of said groups G1 and G2,
said groups G1 and G2 respectively being a nitrone and a strained cycloalkynyl radical or vice versa.
